# EUROPÄISCHE PATENTSCHRIFT

(11) **EP 2 814 408 B1**
(45) Veröffentlichungstag und Bekanntmachung des Hinweises auf die Patenterteilung: **08.08.2018**
(21) Anmeldenummer: 13706158.6
(22) Anmeldetag: 09.01.2013
(51) Int. Cl.: A61B 17/3201, A61B 17/32, A61F 9/007, A61B 17/29

(54) **MEDIZINISCHES INSTRUMENT**
MEDICAL INSTRUMENT
INSTRUMENT MÉDICAL

(30) Priorität: 17.02.2012 DE 102012202434
(43) Veröffentlichungstag der Anmeldung: 24.12.2014
(73) Patentinhaber: Geuder AG, 69126 Heidelberg (DE)
(72) Erfinder: EDER, Juergen, 69251 Gaiberg (DE)
(74) Vertreter: Patent- und Rechtsanwälte Ullrich & Naumann
(86) Internationale Anmeldenummer: PCT/DE2013/200000
(87) Internationale Veröffentlichungsnummer: WO 2013/120491

(56) Entgegenhaltungen:
- DE-A1-102009 033 015
- US-A- 5 352 235
- US-A- 5 797 939
- US-A- 5 984 939
- US-A- 6 086 606
- US-A- 6 099 550
- US-A1- 2010 305 564

## Beschreibung

Die Erfindung betrifft ein medizinisches Instrument, insbesondere Schere, Zange oder dgl., zur Anwendung in der Ophthalmologie.

Medizinische Instrumente der in Rede stehenden Art sind seit Jahren aus der Praxis bekannt. Insbesondere bei chirurgischen Eingriffen muss Gewebe beispielsweise zerschnitten, abgeklemmt, gehalten oder weggezogen werden. Des Weiteren muss der Operateur auch andere Materialien, beispielsweise, Nahtmaterial, Tupfer, Nadeln, Verbänden, Tamponaden etc. greifen und/oder zertrennen. Dazu sind Scheren und Zangen in den unterschiedlichsten Ausführungsformen vorbekannt. Bei Scheren zur Anwendung in der ophthalmologischen Chirurgie werden die Klingen durch eine relativ zu den Klingen verschiebbare Hülse aufeinander zu bzw. voneinander weg bewegt. Dazu ist der Körper der Schere von der Hülse umgeben, wobei die Klingen zumindest teilweise aus der Hülse herausragen. Ähnliche Konstruktionen existieren, um Material zu greifen oder wegzuziehen, wobei bei diesen Instrumenten Greifbacken einer Zange durch eine verschiebbare Hülse gegeneinander bewegbar sind.

An dieser Stelle sei angemerkt, dass im Folgenden zur Vereinfachung lediglich von Gewebe die Rede ist. Darunter sind sowohl die voranstehend genannten Materialien als auch Gewebe, insbesondere Gewebe des menschlichen oder tierischen Auges, zu verstehen.

Die in Rede stehenden Instrumente müssen minimale Abmessungen aufweisen, so dass es sich um sehr filigrane und in der Herstellung aufwendige Instrumente handelt. Dies führt dazu, dass beispielsweise beim Schneiden durch den vom Gewebe ausgeübten Gegendruck die Klingenblätter der Klingen nicht mehr aneinander gleiten sondern voneinander abgespreizt werden.

Insbesondere beim Zerschneiden von härterem Gewebe wird dieses daher gequetscht, was zu erheblichen, mitunter irreparablen Verletzungen des Auges führen kann. Die exakte Schnittführung des Instruments ist nicht mehr gewährleistet. Des Weiteren wird das Gewebe während des Schnitts derart verschoben, dass die Zertrennung des Gewebes nicht mehr an dem von dem Operateur gewählten Punkt erfolgt.

Beim Abspreizen der Greifbacken ist problematisch, dass nicht genug Druck auf das Gewebe ausgeübt wird, um dieses sicher zu fassen. Beispielsweise beim Nähen von Gewebe muss der Operateur jedoch mitunter größeren Druck auf das Nahtmaterial ausüben, um einen chirurgischen Knoten zu knüpfen. Die aus der Praxis bekannten Instrumente eignen sich hierfür nicht.

DE 10 2009 033015 A1 offenbart ein medizinisches Instrument zur Anwendung in der Ophthalmologie, mit einem einen Wirkbereich und einen Betätigungsbereich aufweisenden Werkzeug und einer Hülse, wobei der Wirkbereich zwei gegeneinander bewegbare, zusammenwirkende Wirkelemente aufweist, wobei die Hülse das Werkzeug zumindest im Betätigungsbereich ganz oder teilweise umgibt, wobei die Wirkelemente durch Verschieben der Hülse relativ zum Werkzeug gegeneinander bewegbar sind.

Ein präzises und sicheres Arbeiten ist mit den aus der Praxis bekannten Instrumenten daher nur bedingt möglich. Insbesondere die sichere Handhabung von härterem Gewebe, das einen höheren Gegendruck auf die Schneiden bzw. Greifbacken ausübt, ist mit den bekannten Instrumenten nicht möglich.

Der vorliegenden Erfindung liegt daher die Aufgabe zugrunde, ein medizinisches Instrument der eingangs genannten Art derart auszugestalten und weiterzubilden, dass mit konstruktiv einfachen Mitteln eine sichere und präzise Funktion des Instruments gewährleistet ist.

Erfindungsgemäß wird die voranstehende Aufgabe durch die Merkmale des Patentanspruchs 1 gelöst. Damit ist ein medizinisches Instrument angegeben, mit einem einen Wirkbereich und einen Betätigungsbereich aufweisenden Werkzeug und einer Hülse, wobei der Wirkbereich zwei gegeneinander bewegbare, zusammenwirkende Wirkelemente aufweist, wobei die Hülse das Werkzeug zumindest im Betätigungsbereich ganz oder teilweise umgibt, wobei die Wirkelemente durch Verschieben der Hülse relativ zum Werkzeug gegeneinander bewegbar sind und wobei an der Hülse Ausnehmungen zur Führung der Wirkelemente ausgebildet sind.

In erfindungsgemäßer Weise ist erkannt worden, dass die zugrundeliegende Aufgabe durch eine geschickte Ausgestaltung des Werkzeugs und der das Werkzeug umgebenden Hülse gelöst werden kann. Dazu umfasst das Werkzeug einen Wirkbereich und einen Betätigungsbereich, wobei der Wirkbereich zwei gegeneinander bewegbare, zusammenwirkende Wirkelemente aufweist. Die Hülse umgibt das Werkzeug zumindest im Betätigungsbereich ganz oder teilweise, wobei die Wirkelemente durch ein Verschieben der Hülse relativ zum Werkzeug gegeneinander bewegbar sind. In weiter erfindungsgemäßer Weise ist erkannt worden, dass die präzise Funktion des medizinischen Instruments auf verblüffend einfache Weise gewährleistet ist, wenn an der Hülse Ausnehmungen zur Führung der Wirkelemente ausgebildet sind. Ein Abspreizen der Wirkelemente bei erhöhtem Gegendruck, beispielsweise während des Zertrennens oder Greifens von härterem Gewebe, wird dadurch effektiv vermieden. Die Wirkelemente sind in den Ausnehmungen exakt geführt. Ungewollte Bewegungen der Wirkelemente sind durch die Ausnehmungen gehemmt. Für den Operateur ist daher stets gewährleistet, dass das Instrument präzise an dem von ihm gewünschten Punkt wirkt. Aufgrund der einfachen Konstruktion ist die Herstellung des erfindungsgemäßen medizinischen Handgeräts preiswert und von geringem Aufwand.

Es sei ausdrücklich darauf hingewiesen, dass der Übergang zwischen Wirkbereich und Betätigungsbereich fließend ist. Die Hülse kann das Werkzeug auch - zumindest teilweise - im Wirkbereich umgeben. Maßgeblich ist, dass die Hülse derart mit dem Werkzeug zusammenwirkt, dass die Wirkelemente durch eine Verschiebung der Hülse relativ zu dem Werkzeug gegeneinander bewegbar sind. Dabei kann die Hülse das Werkzeug im Betätigungsbereich und/oder im Wirkbereich und/oder im Übergang zwischen Betätigungsbereich und Wirkbereich umgeben.

Um eine möglichst gleichmäßige Bewegung der Wirkelemente gegeneinander zu gewährleisten, weist die Hülse im Bereich ihrer Ausnehmungen Schrägen auf, auf denen die Wirkelemente gleiten. Die Schrägen können im inneren Bereich der Hülse - beispielsweise als Fase - ausgebildet sein. Während der Herstellung des Instruments vereinfachen die Schrägen die Einführung des Werkzeugs in die Hülse. Des Weiteren gleiten die Wirkelemente besser auf den Schrägen, so dass die Bewegung der Wirkelemente flüssiger bzw. gleichmäßiger ausführbar ist.

In besonders vorteilhafter Weise sind die Ausnehmungen derart ausgebildet, dass die Wirkelemente im geöffneten und im geschlossenen Zustand von den Ausnehmungen geführt sind. Durch diese Konstruktion ist gewährleistet, dass die Wirkelemente unabhängig von ihrer Positionierung relativ zur Hülse, stets in den Ausnehmungen geführt sind. Ungewollte Ausweichbewegungen der Wirkelemente, beispielsweise beim Schneiden oder Greifen von härterem Gewebe, sind durch die Ausnehmungen stets gehemmt.

Zur weiteren Optimierung der Handhabung für den Operateur kann die Hülse an ihrem distalen Ende Abschrägungen aufweisen. Aufgrund des abgeschrägten distalen Endes der Hülse versperrt diese dem Operateur nicht die Sicht auf die Eingriffsstelle.

In vorteilhafter Weise ist an den Wirkelementen jeweils eine linear oder kurvenförmig verlaufende Gleitfläche ausgebildet, mit der die Wirkelemente auf den Ausnehmungen der Hülse gleiten. Je nach Ausgestaltung der Gleitflächen wird ein anderer Bewegungsverlauf der Wirkelemente erreicht. Bei gleicher relativer Geschwindigkeit zwischen Hülse und Werkzeug ergibt sich in Abhängigkeit von der Ausbildung der Gleitflächen eine unterschiedliche Geschwindigkeit, mit der sich die Wirkelemente gegeneinander bewegen. Über die Ausgestaltung der Gleitflächen - linear oder kurvenförmig - kann auch Einfluss darauf genommen werden, wie weit sich die Wirkelemente bei einer relativen Verschiebung der Hülse zu dem Werkzeug gegeneinander bewegen. Die Gleitflächen können beispielsweise derart ausgeformt sein, dass sich die Wirkelemente bei einer großen Verschiebung der Hülse relativ zu dem Instrument nur geringfügig gegeneinander bewegen. Die Gleitflächen können dadurch auf verschiedene Operationstechniken oder auf Vorlieben des Operateurs angepasst sein. Der Operateur kann somit ein Instrument auswählen, dessen Wirkelemente für den jeweiligen Eingriff das optimale Bewegungsverhalten aufweisen.

Um die Verschiebung zwischen Hülse und Instrument zu begrenzen, kann an mindestens einem Wirkelement ein Anschlag ausgebildet sein. Durch den Anschlag ist es möglich, die Bewegung der Wirkelemente gegeneinander ab einem bestimmten Punkt zu hemmen.

In vorteilhafter Weise sind die Wirkelemente als Klingen einer Schere ausgebildet. Durch die Verschiebung der Hülse relativ zum Werkzeug werden die Klingen gegeneinander bewegt, so dass die Klingen eine Schneidbewegung ausführen. Da die Klingen in den Ausnehmungen der Hülse geführt sind, wird ein Abspreizen der Klingenblätter beim Schneiden von härterem Gewebe weitgehend verhindert.

In besonders vorteilhafter Weise können die Ausnehmungen derart dimensioniert sein, dass die Klingen mit ihren Klingenblättern gegeneinander gedrückt sind. Die Klingen werden dadurch auf Vorspannung gehalten. Diese Konstruktion stellt eine weitere Maßnahme zur Vermeidung eines Abspreizens der Klingenblätter dar. Ein weiterer Vorteil liegt darin, dass die Klingen stets in einer präzisen Position gehalten sind. Des Weiteren ist denkbar, dass das Werkzeug in Form einer Bügelschere ausgeführt ist.

In weiter vorteilhafter Weise können die Wirkelemente als Greifbacken einer Zange ausgeführt sein. Durch die relative Verschiebung zwischen Hülse und Werkzeug sind die Greifbacken gegeneinander bewegbar. Das präzise Greifen von Gewebe ist durch die Führung der Greifbacken in den Ausnehmungen der Hülse auch bei größerem Gegendruck gewährleistet.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematische Darstellung ein Ausführungsbeispiel eines erfindungsgemäßen medizinischen Instruments in einer perspektivischen Ansicht,
- Fig. 2: in einer schematischen Darstellung das medizinische Instrument aus Fig. 1 in einer Draufsicht und
- Fig. 3: in einer schematischen Darstellung das erfindungsgemäße medizinische Instrument aus Fig. 1 in einer Seitenansicht.

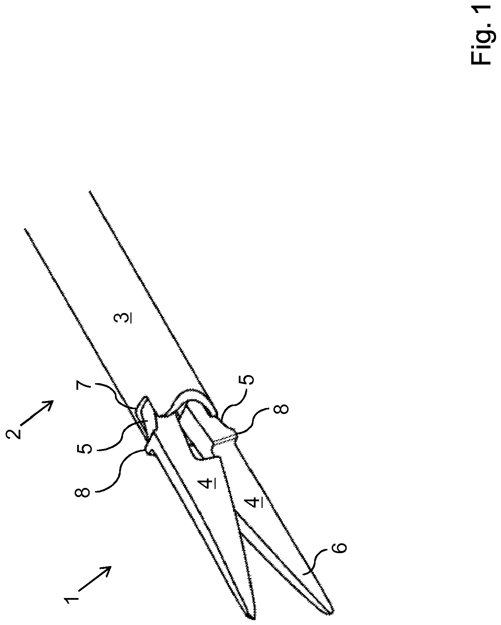

Fig. 1 zeigt in einer schematischen Ansicht ein Ausführungsbeispiel eines erfindungsgemäßen medizinischen Instruments. Das Instrument ist als Schere zur Anwendung in der ophthalmologischen Chirurgie ausgebildet. Das Instrument weist ein Werkzeug auf. Das Werkzeug umfasst einen Wirkbereich 1 und einen Betätigungsbereich 2, wobei der Betätigungsbereich 2 von einer Hülse 3 umgeben ist. In Fig.1 ist daher lediglich der Wirkbereich 1 sichtbar, der Betätigungsbereich 2 ist innerhalb der Hülse 3 angeordnet. Der Wirkbereich 1 weist zwei gegeneinander bewegbare Wirkelemente 4 auf. Aus Fig.1 geht deutlich hervor, dass der Übergang zwischen Betätigungsbereich 1 und Wirkbereich 2 nicht eindeutig definierbar sondern vielmehr fließend ist.

Die Wirkelemente 4 sind als Klingen einer Schere ausgeführt. Die Wirkelemente 4 können auch als Greifbacken einer Zange ausgebildet sein, so dass das medizinische Instrument zum Greifen von Gewebe dient.

In Fig. 1 ist das Werkzeug im geöffneten Zustand gezeigt, d.h. die Klingen 4 der Schere befinden sich in der Offenstellung. Die Klingen 4 können in Richtung ihrer Offenstellung oder entgegen ihrer Offenstellung vorgespannt sein. Die Vorspannung kann beispielsweise über eine Feder oder eine beliebige andere Konstruktion erfolgen. Des Weiteren ist denkbar, dass das Werkzeug entsprechend einer Bügelschere ausgeführt ist, so dass die Klingen 4 aufgrund ihrer Eigenspannung in Richtung ihrer Offenstellung vorgespannt sind.

Bei einer Verschiebung zwischen Hülse 3 und Werkzeug gleiten die Klingen 4 mit ihren Gleitflächen 5 auf der Innenseite der Hülse 3 entlang, wodurch die Klingen 4 gegeneinander bewegt werden. Werden die Hülse 3 und das Werkzeug relativ zueinander verschoben, so führen die Klingen 4 eine Schneidbewegung aus.

Während sich die Klingen 4 gegeneinander bewegen gleiten sie mit den Klingenblättern 6 aufeinander. Um ein Abspreizen der Klingenblätter 6, wie es zum Beispiel beim Schneiden von härterem Gewebe auftreten kann, zu vermeiden, sind die Klingen 4 in Ausnehmungen 7 der Hülse 3 geführt. Die Ausnehmungen 7 führen die Klingen 4 unabhängig von der Position der Hülse 3. Durch die Ausnehmungen 7 wird verhindert, dass die Klingen 4 bei starkem Gegendruck voneinander abgespreizt werden. Die Ausnehmungen 7 sind derart ausgebildet, dass die Klingenblätter 6 zumindest geringfügig gegeneinander gedrückt sind. Durch den Druck, den die Klingenblätter 6 aufeinander ausüben, wird ein Abspreizen der Klingenblätter 6 während des Schneidens vermieden.

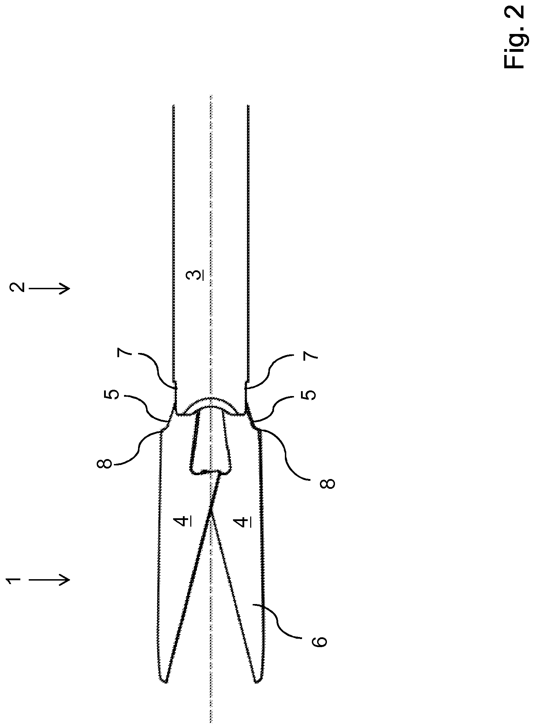

In Fig. 2 ist das Ausführungsbeispiel eines erfindungsgemäßen Instruments aus Fig. 1 in einer Draufsicht gezeigt. Aus Fig. 2 geht deutlich hervor, dass die Klingen 4 jeweils einen Anschlag 8 aufweisen. Die Anschläge 8 dienen dazu, die Verschiebung zwischen Hülse 3 und Werkzeug zu begrenzen. Hierdurch kann exakt definiert werden, wie weit die Klingen 4 gegeneinander bewegt werden können. Sind die Wirkelemente 4 als Greifbacken ausgebildet, so ist über den Anschlag 8 bzw. die Anschläge 8 einstellbar, ob sich die Greifbacken in der geschlossenen Position berühren.

Des Weiteren ist in Fig. 2 erkennbar, dass die Gleitflächen 5 kurvenförmig ausgeführt sind. Der Bewegungsablauf der Klingen 4 wird bei einer Verschiebung zwischen Hülse 3 und Werkzeug durch die Krümmung der Gleitflächen 5 definiert. Je nach Ausgestaltung der Gleitflächen 5 bewegen sich die Klingen 4 bei einer Verschiebung der Hülse 3 relativ zu dem Werkzeug schneller oder langsamer.

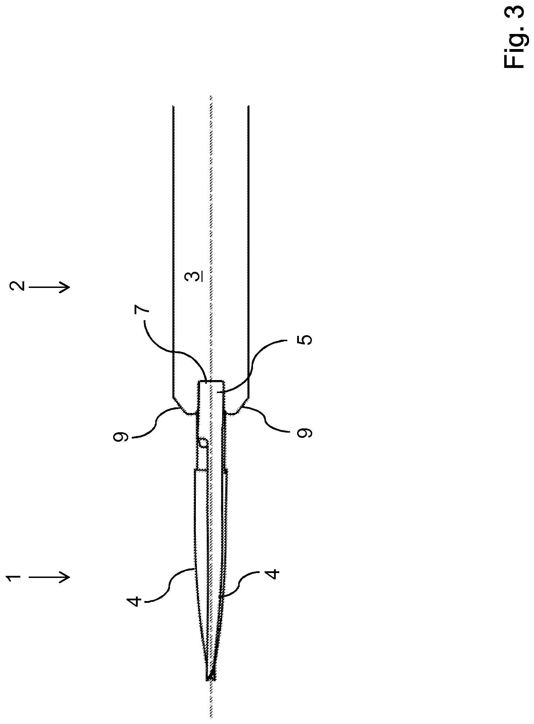

Fig. 3 zeigt das Ausführungsbeispiel aus Fig. 1 in einer Seitenansicht. Dabei ist deutlich zu erkennen, dass die Hülse 3 an ihrem distalen Ende Abschrägungen 9 aufweist. Durch die abgeschrägte Ausführung des distalen Endes der Hülse 3 versperrt diese dem Operateur nicht die Sicht auf das Eingriffsfeld. Zur Vermeidung von Wiederholungen sei in Bezug auf die weitere Ausgestaltung des Instruments auf die Beschreibung der Fig. 1 und 2 verwiesen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel des erfindungsgemäßen medizinischen Instruments lediglich zur Erörterung der beanspruchten Lehre dient, diese jedoch nicht auf das Ausführungsbeispiel einschränkt.

### Bezugszeichenliste

- 1: Wirkbereich
- 2: Betätigungsbereich
- 3: Hülse
- 4: Wirkelement
- 5: Gleitfläche
- 6: Klingenblatt
- 7: Ausnehmung
- 8: Anschlag
- 9: Abschrägung

## Patentansprüche

1. Medizinisches Instrument zur Anwendung in der Ophthalmologie, mit einem einen Wirkbereich (1) und einen Betätigungsbereich (2) aufweisenden Werkzeug und einer Hülse (3), wobei der Wirkbereich (1) zwei gegeneinander bewegbare, zusammenwirkende Wirkelemente (4) aufweist, wobei die Hülse (3) das Werkzeug zumindest im Betätigungsbereich (2) ganz oder teilweise umgibt, wobei die Wirkelemente (4) durch Verschieben der Hülse (3) relativ zum Werkzeug gegeneinander bewegbar sind,
**dadurch gekennzeichnet, dass** am distalen Ende der Hülse (3) Ausnehmungen (7) zur Führung der Wirkelemente (4) ausgebildet sind und dass die Hülse (3) im Bereich ihrer Ausnehmungen (7) Schrägen aufweist, auf denen die Wirkelemente (4) gleiten.

2. Medizinisches Instrument nach Anspruch 1, **dadurch gekennzeichnet, dass** es sich um eine Schere oder um eine Zange handelt.

3. Medizinisches Instrument nach Anspruch 1 oder 2, **dadurch gekennzeichnet, dass** die Ausnehmungen (7) derart ausgebildet sind, dass die Wirkelemente (4) im geöffneten und im geschlossenen Zustand von den Ausnehmungen (7) geführt sind.

4. Medizinisches Instrument nach einem der Ansprüche 1 bis 3, **dadurch gekennzeichnet, dass** die Hülse (3) an ihrem distalen Ende Abschrägungen (9) aufweist, um so eine bessere Sicht auf eine Eingriffsstelle zu gewährleisten.

5. Medizinisches Instrument nach einem der Ansprüche 1 bis 4, **dadurch gekennzeichnet, dass** die Wirkelemente (4) jeweils eine linear oder kurvenförmig verlaufende Gleitfläche (5) aufweisen, mit der sie in den Ausnehmungen (7) gleiten.

6. Medizinisches Instrument nach einem der Ansprüche 1 bis 5, **dadurch gekennzeichnet, dass** an mindestens einem Wirkelement (4) ein Anschlag (8) ausgebildet ist, um eine Verschiebung der Hülse (3) zu begrenzen.

7. Medizinisches Instrument nach einem der Ansprüche 1 bis 6, **dadurch gekennzeichnet, dass** die Wirkelemente (4) als Klingen einer Schere ausgeführt sind.

8. Medizinisches Instrument nach Anspruch 7, **dadurch gekennzeichnet, dass** die Ausnehmungen (7) derart dimensioniert sind, dass die Klingen (4) mit ihren Klingenblättern (6) gegeneinander gedrückt sind.

9. Medizinisches Instrument nach Anspruch 7 oder 8, **dadurch gekennzeichnet, dass** das Werkzeug in Form einer Bügelschere ausgeführt ist.

10. Medizinisches Instrument nach einem der Ansprüche 1 bis 6, **dadurch gekennzeichnet, dass** die Wirkelemente (4) als Greifbacken einer Zange ausgeführt sind.

## Claims

1. Medical instrument for use in ophthalmology, having a tool which has an active region (1) and an actuation region (2) and having a sleeve (3), wherein the active region (1) has two cooperating active elements (4) which can be moved against each other, wherein the sleeve (3) completely or partially surrounds the tool at least in the actuation region (2), wherein the active elements (4) can be moved against each other by displacing the sleeve (3) relative to the tool, **characterised in that** at the distal end of the sleeve (3) recesses (7) are constructed for guiding the active elements (4) and **in that** the sleeve (3) has in the region of the recesses (7) thereof inclinations, on which the active elements (4) slide.

2. Medical instrument according to claim 1, **characterised in that** it is a set of scissors or tongs.

3. Medical instrument according to claim 1 or 2, **characterised in that** the recesses (7) are constructed in such a manner that the active elements (4) in the open and closed state are guided by the recesses (7).

4. Medical instrument according to any one of claims 1 to 3, **characterised in that** the sleeve (3) has at the distal end thereof chamfered portions (9) in order to thus ensure a better view of an engagement location.

5. Medical instrument according to any one of claims 1 to 4, **characterised in that** the active elements (4) each have a sliding face (5) which extends in a linear or curved manner and by means of which they slide in the recesses (7).

6. Medical instrument according to any one of claims 1 to 5, **characterised in that** a stop (8) is constructed on at least one active element (4) in order to limit a displacement of the sleeve (3).

7. Medical instrument according to any one of claims 1 to 6, **characterised in that** the active elements (4) are constructed as blades of a set of scissors.

8. Medical instrument according to claim 7, **characterised in that** the recesses (7) are sized in such a manner that the blades (4) are pressed against each other with the blade plates (6) thereof.

9. Medical instrument according to claim 7 or 8, **characterised in that** the tool is constructed in the form of a set of curved scissors.

10. Medical instrument according to any one of claims 1 to 6, **characterised in that** the active elements (4) are constructed as gripping jaws of a set of tongs.

## Revendications

1. Instrument médical destiné à être utilisé en ophtalmologie, avec un outil comprenant une zone d'action (1) et une zone d'actionnement (2) et un manchon (3), la zone d'action (1) comprenant deux éléments actifs (4) mobiles et interagissant entre eux, le manchon (3) entourant entièrement ou partiellement l'outil au moins dans la zone d'actionnement (2), les zones d'action (4) étant mobiles entre elles par déplacement du manchon (3) par rapport à l'outil,
**caractérisé en ce que**, sur l'extrémité distale du manchon (3) sont réalisées des évidements (7) pour le guidage des éléments actifs (4) et **en ce que** le manchon (3) comprend au niveau de ses évidements (7) des chanfreins sur lesquels les éléments actifs (4) glissent.

2. Instrument médical selon la revendication 1, **caractérisé en ce qu'**il s'agit de ciseaux ou d'une pince.

3. Instrument médical selon la revendication 1 ou 2, **caractérisé en ce que** les évidements (7) sont conçus de façon à ce que les éléments actifs (4) sont guidés par les évidements (7) dans l'état ouvert et dans l'état fermé.

4. Instrument médical selon l'une des revendications 1 à 3, **caractérisé en ce que** le manchon (3) comprend, au niveau de son extrémité distale, des chanfreins (9), afin de garantir une meilleure vue sur un point d'intervention.

5. Instrument médical selon l'une des revendications 1 à 4, **caractérisé en ce que** les éléments actifs (4) comprennent chacun une surface de glissement (5) linéaire ou incurvée, avec laquelle ils glissent dans les évidements (7).

6. Instrument médical selon l'une des revendications 1 à 5, **caractérisé en ce que**, sur au moins un élément actif (4), une butée (8) est prévue pour limiter un déplacement du manchon (3).

7. Instrument médical selon l'une des revendications 1 à 6, **caractérisé en ce que** les éléments actifs (4) sont conçus comme des lames de ciseaux.

8. Instrument médical selon la revendication 7, **caractérisé en ce que** les évidements (7) sont dimensionnés de façon à ce que les lames (4) sont comprimées entre elles avec leurs plats de lames (6).

9. Instrument médical selon la revendication 7 ou 8, **caractérisé en ce que** l'outil est conçu sous la forme de ciseaux à étrier.

10. Instrument médical selon l'une des revendications 1 à 6, **caractérisé en ce que** les éléments actifs (4) sont conçus comme des mâchoires d'une pince.
